# EUROPEAN PATENT APPLICATION

(11) **EP 2 009 080 A1**
(43) Date of publication of application: **31.12.2008**
(21) Application number: 07741000.9
(22) Date of filing: 04.04.2007
(51) Int. Cl.: C10L 3/10, B01D 19/00, B01D 53/14, B01D 61/00, B01D 63/02, B01D 71/26

(54) **METHOD FOR SEPARATION OF METHANE, METHANE SEPARATOR, AND METHANE UTILIZATION SYSTEM**

(30) Priority: 04.04.2006 JP 2006103665
(71) Applicant: Taiyo Nippon Sanso Corporation, Tokyo 142-8558 (JP); Research Institute Of Innovative Technology For The Earth, Kizugawa-shi, Kyoto 619-0292 (JP)
(72) Inventor: TOMIOKA, Takafumi, Shinagawa-ku Tokyo 142-8558 (JP); ABE, Toshiyuki, Tokyo 142-8558 (JP); SAKAI, Toru, Tokyo 142-8558 (JP); MANO, Hiroshi, Kizugawa-shi Kyoto 619-0292 (JP); OKABE, Kazuhiro, Kizugawa-shi Kyoto 619-0292 (JP)
(74) Representative: Waldren, Robin Michael
(86) International application number: PCT/JP2007/057564
(87) International publication number: WO 2007/116908

(57) **Abstract**

A methane separation method of the present invention at least includes: mixing the biogas and an absorbing liquid that absorbs carbon dioxide in a mixer so as to form a mixed fluid of a gas-liquid mixed phase; introducing the mixed fluid into a first gas/liquid separator so as to separate the mixed fluid through gas/liquid separation into methane and a CO₂-absorbed liquid formed due to an absorption of the carbon dioxide by the absorbing liquid; recovering methane separated in the first gas/liquid separator; and supplying the CO₂-absorbed liquid through a supply port of a membrane module comprised of a container and a plurality of hollow fiber permeable membranes built therein to inside of the membranes so as to make the CO₂-absorbed liquid permeate the permeable membranes, and lowering a pressure outside the permeable membranes to a level lower than that inside the permeable membranes.

## Description

### TECHNICAL FIELD

The present invention relates to a methane separation method that separates methane from biogases such as a natural gas that has methane as its major component and is generated from underground due to the anaerobic fermentation by organisms, an underground fermentation gas produced by a natural anaerobic fermentation due to an underground burial of industrial and domestic wastes, and an artificial fermentation gas generated artificially and discharged from an anaerobic fermentation process, a methane separation apparatus that carries out the method, and a methane utilization system that is capable of supplying the separated methane to the energy market.
Priority is claimed on Japanese Patent Application No. 2006-103665, filed April 4, 2006, the content of which is incorporated herein by reference.

### BACKGROUND ART

There are cases where a large amount of carbon dioxide and water that cannot be used as a heat energy source are contained in the gases constituted by having methane as their major component such as a natural gas that has methane as its major component and is generated from underground due to the anaerobic fermentation by organisms, an underground fermentation gas produced by a natural anaerobic fermentation due to an underground burial of industrial and domestic wastes, an artificial fermentation gas generated artificially and discharged from an anaerobic fermentation process, and a COG gas generated during the coke production. In order to use these gases as a good quality heat energy source and also as a fuel, it is necessary to remove those contained in a mixed gas that cannot be used as a fuel such as carbon dioxide and water and to enhance the purity of methane.

As the methods for enhancing the purity of a specific gas component in these mixed gases, a low temperature processing method that distills and separates a mixed gas under low temperature conditions, a chemical absorption method, a dry membrane separation process using a gas separation membrane, a pressure swing adsorption (PSA) process, a membrane/absorption hybrid method, and the like are known. In the low temperature processing method, the separation process involves the comings and goings of heat and it is not preferable from an economical viewpoint since an apparatus will be complex and also will be large in size if highly pure methane were to be obtained efficiently.

In addition, in the conventional chemical absorption method, an apparatus will have an absorption column, in which an absorbing liquid absorbs a target gas for separation, and a regeneration column, in which the separated gas components are released from the absorbing liquid, and the absorbing liquid circulates between the absorption column and the regeneration column so that the target gas for separation is separated continuously. Accordingly, there has been a problem of increase in the initial cost as well as the operation cost for gas separation due to the absorption column for efficiently bringing the absorbing liquid into contact with the target gas and a large amount of heating energy required for the release. Moreover, the requirement for a large amount of water for purifying biogases has also been a problem, although there is a so-called carbonate absorption process available which exploits the effect that carbon dioxide dissolves in water and uses high pressure water.

On the other hand, there are advantages with the dry membrane separation process and the PSA process such as the absence of comings and goings of heat, capability of the methane separation with low energy, operability at normal temperatures, and the possible construction of apparatuses having simple structures with reduced sizes. However, since the differences in the permeation rates among the membranes are exploited in the dry membrane separation process, it is necessary to increase the number of steps in the membrane module in order to obtain highly pure methane resulting in higher cost, which has been a problem. As shown in Patent Documents 1 and 2, the PSA process uses activated carbon, natural or synthetic zeolite, silica gel and activated alumina, molecular sieving carbon (MSC), or the like as an adsorbent that readily adsorbs carbon dioxide, and exploits the fact that the amount of adsorption differs depending on pressure and temperature.

However, in the PSA process, a pressure range in which an apparatus is operated needs to be set within a wide range of -90 KPaG to 0.7 MPaG in order to obtain highly pure methane, and thus the resulting high power cost is a problem. In addition, recovery rate needs to be sacrificed in order to obtain highly pure methane which leads to the generation of a large amount of exhaust gas containing methane. Accordingly, it will be necessary to install a combustion facility and the like to safely treat methane that is flammable resulting in a high cost for the separation process. Moreover, even if methane can be released in the air safely, it will be a great disadvantage to release methane that has a high global warming potential into the atmosphere when considering the increase in the awareness of global environmental issues in recent years.

Accordingly, a membrane/absorption hybrid method where the membrane separation process and the chemical absorption method coexist simultaneously to bring about synergistic effects therefrom has been drawing attention and being studied (refer to Patent Document 3 and Non-patent Document 1). In this method, a gas containing carbon dioxide (CO₂) and an absorbing liquid are supplied in one side of a membrane so that carbon dioxide is absorbed in the absorbing liquid to pass through the membrane and carbon dioxide is released from the absorbing liquid that passed through the membrane by reducing the pressure in the other side of the membrane.

For this reason, only carbon dioxide that is unnecessary is selectively separated in the absorbing liquid, and thus can be separated from the combustible components. Hence, the recovery rate of methane that is a combustible component can be improved. In addition, there is no need to provide a combustion facility for treating exhaust gases since there is no combustible component in the exhaust gases, and thus the exhaust gases can be treated at an extremely low cost, which is an advantage. Moreover, according to this method, the heat transfer between inside and outside the membrane takes place effectively since the reaction absorbing carbon dioxide is an exothermic reaction and the reaction releasing carbon dioxide is an endothermic reaction. Accordingly, it will be possible to separate carbon dioxide extremely efficiently while balancing the heat balance of absorption and release processes. Moreover, a continuous methane separation can be carried out by circulating and reusing the absorbing liquid. Therefore, it will be possible to separate highly pure methane from biogases at a lower operation cost than those of the conventional chemical absorption method, the dry membrane separation process, and the PSA process by applying the membrane/absorption hybrid method to biogases.

However, when applying the membrane/absorption hybrid method to biogases, a gas-liquid mixed phase of methane that does not dissolve in an absorbing liquid and the absorbing liquid is generated inside a permeable membrane since a target gas for separation containing gases to be separated such as carbon dioxide and methane is supplied together with the absorbing liquid to, for example, a membrane module, resulting in a decline of methane separation efficiency, which is a problem. Moreover, since high concentration of carbon dioxide is contained in biogases, satisfactory release of the carbon dioxide absorbed in the absorbing liquid that permeated the permeable membrane of the membrane module is not achieved and the absorbing liquid returns to an absorbing-liquid circulation system without being regenerated satisfactorily, and thus a methane purification efficiency declines, which is also a problem.

[Patent Document 1]
   Japanese Unexamined Patent Application, First Publication No. 2001-293340
[Patent Document 2]
   Japanese Unexamined Patent Application, First Publication No. 2003-204853
[Patent Document 3]
   Japanese Unexamined Patent Application, First Publication No. 2005-270814
[Non-patent Document 1]
   Masaaki Teramoto, Nobuaki Ohnishi, Nao Takeuchi, Satoru Kitada, Hideto Matsuyama, Norifumi Matsumiya, Hiroshi Mano: "Separation and enrichment of carbon dioxide by capillary membrane module with permeation of carrier solution"; Separation and Purification Technology 30 (2003) 215-227

### DISCLOSURE OF INVENTION

### PROBLEMS TO BE SOLVED BY THE INVENTION

Accordingly, an object of the present invention is to provide a methane separation method capable of separating and purifying methane at high efficiency from biogases having methane as their major component and containing high concentration of carbon dioxide, a methane separation apparatus that carries out the method, and a methane utilization system capable of supplying methane to the energy market like the already available fossil fuels such as petroleum.

### MEANS FOR SOLVING THE PROBLEMS

The present invention is made in order to solve the abovementioned problems and the methane separation method and the methane separation apparatus according to the present invention are characterized by separating methane from biogases containing methane and carbon dioxide as their components due to the following steps.
That is, a first embodiment of the present invention is
a methane separation method at least including the steps of:
mixing the biogas and an absorbing liquid that absorbs carbon dioxide in a mixer so as to form a mixed fluid of a gas-liquid mixed phase;
introducing the mixed fluid into a first gas/liquid separator so as to separate the mixed fluid through gas/liquid separation into methane and a CO₂-absorbed liquid formed due to an absorption of the carbon dioxide by the absorbing liquid;
recovering methane separated in the first gas/liquid separator; and
supplying the CO₂-absorbed liquid through a supply port of a membrane module comprised of a container and a plurality of hollow fiber permeable membranes built therein to inside of the membranes so as to make the CO₂-absorbed liquid permeate the permeable membranes, and lowering a pressure outside the permeable membranes to a level lower than that inside the permeable membranes, thereby releasing the carbon dioxide contained in the CO₂-absorbed liquid to outside of the permeable membranes so as to separate the carbon dioxide, and recovering the absorbing liquid obtained by separating CO₂ from the CO₂-absorbed liquid. Also, a first embodiment of the present invention is a methane separation apparatus that carries out the separation method. In the present embodiment, it is preferable to make the flow rate of an excessive CO₂-absorbed liquid discharged from a discharge port of the membrane module close to zero to the utmost limit.

A second embodiment of the present invention is
a methane separation method further including the steps of:
introducing an excessive CO₂-absorbed liquid discharged from a discharge port of the membrane module to a second gas/liquid separator so as to conduct gas/liquid separation of a trace amount of methane from the excessive CO₂-absorbed liquid;
recovering methane separated in the second gas/liquid separator; and
recovering the excessive CO₂-absorbed liquid. Also, a second embodiment of the present invention is a methane separation apparatus that carries out the separation method. Although most methane is separated by the first gas/liquid separator of the first embodiment, it is configured so that when a trace amount of methane is remaining in the CO₂-absorbed liquid, the CO₂-absorbed liquid discharged from the exhaust port of the membrane module is introduced into the second gas/liquid separator and methane is separated/recovered by the second gas/liquid separator while the excessive CO₂-absorbed liquid is recovered.

A third embodiment of the present invention is a methane separation method and a methane separation apparatus in which a packing density of the permeable membrane in the membrane module is 30% or less. In the present embodiment, the packing density is preferably 20% or less. Note that the packing density of the permeable membrane in the membrane module in the present invention refers to an area occupancy of a permeable membrane, for example, a hollow fiber porous membrane in the section of the membrane module and the hollow portion of the hollow fiber membrane is also included in the occupying area.
It should also be noted that the lower limit for the packing density of a permeable membrane is 5%.

A fourth embodiment of the present invention is a methane separation method and a methane separation apparatus in which the permeable membrane in the membrane module is segmented into small bundles for arrangement, each of the small bundle is arranged so as to retain uncongested space therebetween, and a packing density as a whole is 30% or less.

A fifth embodiment of the present invention is a methane separation method and a methane separation apparatus in which the mixer has an ejector provided in a flow path that communicates to an inlet of the first gas/liquid separator, generates negative pressure by forming a flow of the absorbing liquid inside the flow path, forms the mixed fluid by making the absorbing liquid to suck the biogas, and makes the absorbing liquid to efficiently absorb carbon dioxide. Biogas can be finely dispersed intensively in an absorbing liquid by the ejector and carbon dioxide can be absorbed efficiently by the absorbing liquid.

A sixth embodiment of the present invention is a methane separation method and a methane separation apparatus in which the mixer further includes a packed bubble column, supplies the mixed fluid formed in the ejector to the packed bubble column, and further accelerates an absorption of carbon dioxide in the mixed fluid. The CO₂ absorption can be further intensified by arranging the packed bubble column in series.

A seventh embodiment of the present invention is a methane separation method and a methane separation apparatus in which the absorbing liquid is an aqueous solution of diethanolamine having a concentration of 0.1 to 6 mol/L. In the present embodiment, concentration of the aqueous solution of diethanolamine is preferably 2 to 4 mol/L.

An eighth embodiment of the present invention is a methane separation method and a methane separation apparatus in which a permeation flow rate of the CO₂-absorbed liquid permeating the permeable membrane in the membrane module is 5 to 50 L/m²·min per membrane area. In the present embodiment, the permeation flow rate of the CO₂-absorbed liquid is preferably 20 to 40 L/ m²·min per membrane area.

A ninth embodiment of the present invention is a methane separation method and a methane separation apparatus in which the permeable membrane is formed of polyethylene, and a tenth embodiment of the present invention is a methane separation method and a methane separation apparatus in which an outer surface of the permeable membrane is subjected to a hydrophilic treatment.

An eleventh embodiment of the present invention is a methane utilization system including:
the methane separation apparatus according to the present invention, a methane storage tank, and a methane supply passage, and
in which methane is purified and stored by removing carbon dioxide from at least one of the biogas selected from the group consisting of a natural gas generated from underground due to anaerobic fermentation by organisms and has methane as its major component, an underground fermentation gas produced by a natural anaerobic fermentation due to an underground burial of industrial and domestic wastes, and an artificial fermentation gas discharged from an anaerobic fermentation process that is set up artificially, and the stored methane can be supplied as a fuel.
In addition, a twelfth embodiment of the present invention is a methane utilization system further including a power generation facility that generates electricity using the stored methane as a fuel, and a storage control unit that adjusts a stored amount of purified methane depending on seasons, an operation period, or a time period, and in which the electric power generated by the power generation facility can be supplied to the outside of the system. Moreover, a thirteenth embodiment of the present invention is a methane utilization system further including a carbon dioxide supply facility that enables a hybrid supply of the carbon dioxide that is separated simultaneously when the methane is purified.

### EFFECTS OF THE INVENTION

According to the first embodiment of the present invention, the mixed fluid in a gas-liquid mixed phase formed in the mixer is introduced into the first gas/liquid separator and the separated methane is recovered. Thereafter, the CO₂-absorbed liquid is supplied to the inside of the membrane module, and the pressure outside the permeable membrane is lowered to a level lower than that inside the permeable membrane. Since the separation of carbon dioxide accelerates due to this configuration, methane can be separated and purified at high efficiency from the biogas containing high concentration of carbon dioxide. Therefore, the present invention is capable of reducing power load and membrane module cost and can carry out the separation/concentration of biogases at a low separation cost compared to the already available apparatuses that employ the PSA process, the dry membrane separation process, the chemical absorption method, or the like by adopting the membrane/absorption hybrid method. Since most methane contained in the biogas can be recovered merely by the first gas/liquid separator, simplification of the apparatus configuration and price reduction of the methane separation apparatus will be possible. In addition, power load can be reduced by lowering the flow rate of excessive CO₂-absorbed liquid pouring out from the exhaust port of the membrane module down to a minimum level.
Various mixers capable of dispersing the biogas as minute bubbles in the absorbing liquid can be used as the aforementioned mixer. Specifically, stand alone mixers such as an ejector, a mixer, an aerator, and a packed bubble column of a gas-liquid co-current which is a gas-liquid contacting column where a filler is filled, or combined mixers that combine two or more of the above mixers can be used.

According to the second embodiment of the present invention, further improvements in the methane separation can be achieved since an excessive CO₂-absorbed liquid discharged from the exhaust port of the membrane module is introduced to the second gas/liquid separator and the reseparation/recovering of the remaining trace amount of methane is carried out.

The third and fourth embodiments of the present invention contribute to the improvements in methane separation performance. It has become apparent due to the verification of the present inventors that at the time of carbon dioxide release in the regeneration step of the absorbing liquid, congestion of the membrane module prevents the release. In other words, the packing density of permeable membranes affects the performance of carbon dioxide release in the membrane module. The packing density of hollow fiber permeable membranes in the commercially available membrane modules is 30 to 70% and the interval between adjacent permeable membranes is too packed. For this reason, space between membranes will be covered with liquid membranes when the liquid flow rate is large which makes the release efficiency of carbon dioxide by the reduced pressure more impaired as it approaches the center. As a result, a large membrane area will be required causing a cost increase.
On the other hand, according to the third embodiment of the present invention, the packing density of permeable membranes is 30% or less (preferably 20% or less) which is sparse. Hence, it will be possible to enhance the release properties of carbon dioxide and to separate methane at high efficiency. In addition, according to the fourth embodiment of the present invention, the permeable membrane in the membrane module is segmented into small bundles for arrangement and each of the small bundles is arranged so as to retain uncongested space therebetween, and a packing density as a whole is 30% or less. Hence, it will be possible to make the packing density of permeable membranes sparse, enhance the release properties of carbon dioxide, and separate methane at high efficiency.

According to the fifth embodiment of the present invention, rapid narrowing down of the absorbing liquid generates a high speed flow causing intense negative pressure since at least an ejector is used as the aforementioned mixer, and biogases can be sucked into the absorbing liquid automatically without any power due to this negative pressure. Moreover, minute gas bubbles are formed instantly inside the absorbing liquid and the mixed fluid in a gas-liquid mixed phase is formed efficiently. As a result, a gas/liquid contacting surface area will increase and a large amount of carbon dioxide in the biogas can be absorbed by the absorbing liquid with a shorter contact time and a lesser amount of absorbing liquid compared to the conventional cases. As described so far, an optimum mixed fluid in a gas-liquid mixed phase can be formed and separated through gas/liquid separation and at the same time, the carbon dioxide contained in the biogas at high concentrations can be absorbed by the minimum amount of absorbing liquid. Hence, the methane separation can be carried out at high efficiency without supplying excessive amount of absorbing liquid to the permeable membranes. It will be possible to simplify the apparatus configuration and to reduce price and power cost when the aforementioned mixer is configured only with an ejector. Needless to say, more efficient mixing/absorption can be achieved by adding other mixing units to the ejector.

According to the sixth embodiment of the present invention, a two stage configuration is adopted in which a packed bubble column of a gas-liquid co-current, which is a gas-liquid contacting column where a filler is filled, is connected to the wake side of the ejector in series. Accordingly, a gas-liquid two-phase flow is first formed efficiently by the ejector and the packed bubble column can further accelerate the formation of gas-liquid two-phase flow. Hence, carbon dioxide can almost completely be absorbed by the absorbing liquid and the gas separation thereof from methane can be achieved reliably, and thus methane can almost completely be separated and recovered by the mere first gas/liquid separator.

According to the seventh embodiment of the present invention, the absorbing liquid is an aqueous solution of diethanolamine and its concentration is 0.1 to 6 mol/L (preferably 2 to 4 mol/L). Hence, absorption properties and release properties of carbon dioxide are satisfactory and methane can be separated and purified from biogases at high efficiency.

The eighth embodiment of the present invention contributes to the enhancements in methane concentration. According to the verification by the present inventors, the release efficiency of carbon dioxide does not improve unless the permeation flow rate of the CO₂-absorbed liquid permeating the permeable membranes reaches a predetermined value or more. Therefore, the absorbing liquid circulates within the methane separation apparatus and is supplied to the mixer without being generated satisfactorily. As a result, the carbon dioxide in biogases supplied to the mixer cannot be absorbed satisfactorily. In other words, methane concentration in the gas recovered in the first gas/liquid separator does not increase.
On other hand, according to the present embodiment, the release properties of carbon dioxide improve by making the permeation flow rate of the C₂-absorbed liquid permeating the permeable membrane in the membrane module 5 to 50 L/ m²·min per membrane area (the permeation flow rate of the CO₂-absorbed liquid is preferably 20 to 40 L/ m²·min per membrane area), and thus the concentration of purified methane can be enhanced.

According to the ninth embodiment of the present invention, highly efficient processing of methane separation and purification can be achieved since the permeable membranes are formed of polyethylene. In other words, by using a hydrophobic polyethylene (PE) membrane as the permeable membrane, separation selectivity, permeation rate and long term stability improve compared to those of the conventional permeable membranes. Accordingly, the resistance to, for example, diethanolamine as the absorbing liquid, substantially required permeation amount of the absorbing liquid, and economic efficiency can be improved dramatically. In addition, since the PE membrane is hydrophobic, when the apparatus operation is stopped without filling the membrane module with the absorbing liquid, the wetting of outer surface of the permeable membrane with the absorbing liquid will be unsatisfactory when relaunching the apparatus operation which results in a possible decline of separation efficiency. However, according to the tenth embodiment of the present invention, the problem of possible decline in separation efficiency at the time of launching the apparatus operation can be resolved by chemically subjecting only the outer surface of the permeable membrane to a hydrophilic treatment or by carrying out a physical treatment for enhancing affinity with the absorbing liquid.

Due to the methane utilization system according to the eleventh embodiment of the present invention, since methane is purified and stored based on the highly efficient methane separation method of the present invention and the stored methane can be supplied as a fuel, a methane utilization system capable of economically supplying separated methane at high concentrations can be achieved. In addition, according to the twelfth embodiment of the present invention, a methane utilization system capable of stably supplying the electric power generated by the power generation facility to the outside of the system by efficiently adjusting the stored amount of purified methane can be achieved. Moreover, according to the thirteenth embodiment, a methane utilization system that can supply the carbon dioxide produced as a byproduct during the methane separation/purification can be achieved.

### BRIEF DESCRIPTION OF THE DRAWING

FIG. 1 is a schematic configuration diagram of a methane separation apparatus of a one stage gas/liquid separation system according to the present invention.
FIG. 2 is a schematic configuration diagram of a mixer 5.
FIG. 3 is a schematic configuration diagram of a methane separation apparatus of a two stage gas/liquid separation system according to the present invention.
FIG. 4 is a schematic configuration diagram of a biogas utilization system, which is another embodiment.
FIG. 5 is a comparison chart of concentrations of the concentrated methane obtained by conducting methane separation from the absorbing liquid that absorbed carbon dioxide using 3 different systems.
FIG. 6 is a graph showing the result of studies examining the relationship between the flow rate of liquid permeating a membrane and the recovery rate of methane (CH₄) in Examples 1 to 4.
FIG. 7 is a graph showing the relationship among the flow rate of liquid permeating a membrane, the cost for methane separation, and the pumping power for the absorbing liquid in the methane separation apparatus according to the present embodiment.

### DESCRIPTION OF THE REFERENCE SYMBOLS

3, 9, 20, 31, 64, 65: Supply passage; 5: Mixer; 5a: Ejector; 5b: Packed bubble column; 6a: Delivery passage; 6: Flow path; 13: Discharge passage; 7: First gas/liquid separator; 8, 15, 18, 24, 25, 83: Recovery passage; 10: Membrane module; 11: Permeable membrane; 12, 13a, 22: On/off valve; 14: Second gas/liquid separator; 19: Absorbing liquid storage tank; 21: Exhaust passage; 23: Exhaust pump; 26: Methane recovery unit; 27: Carbon dioxide recovery unit; 28: Supply port; 29: Discharge port; 30: Introduction pump; 32: Nozzle; 50: Membrane/absorption hybrid apparatus; 51: Biogas fermentation tank; 52, 61, 63: Power generator; 53: Hot water storage tank; 54, 55, 58, 60, 62, 71, 74, 76, 78: Flow rate controller; 56, 72: Liquefaction equipment; 57: Liquefied methane storage tank; 59, 75: External supply passage; 66, 70: Supply pump; 67: Calorie controller; 68: Gas concentration meter; 69: LPG tank; 73: Liquefied carbon dioxide storage tank; 77, 79: Facility for greenhouse growth; 80: Power line; 81, 82: Repeater

### BEST MODE FOR CARRYING OUT THE INVENTION

Embodiments of the methane separation method and the methane separation apparatus employing the method according to the present invention will be described below using the drawings.

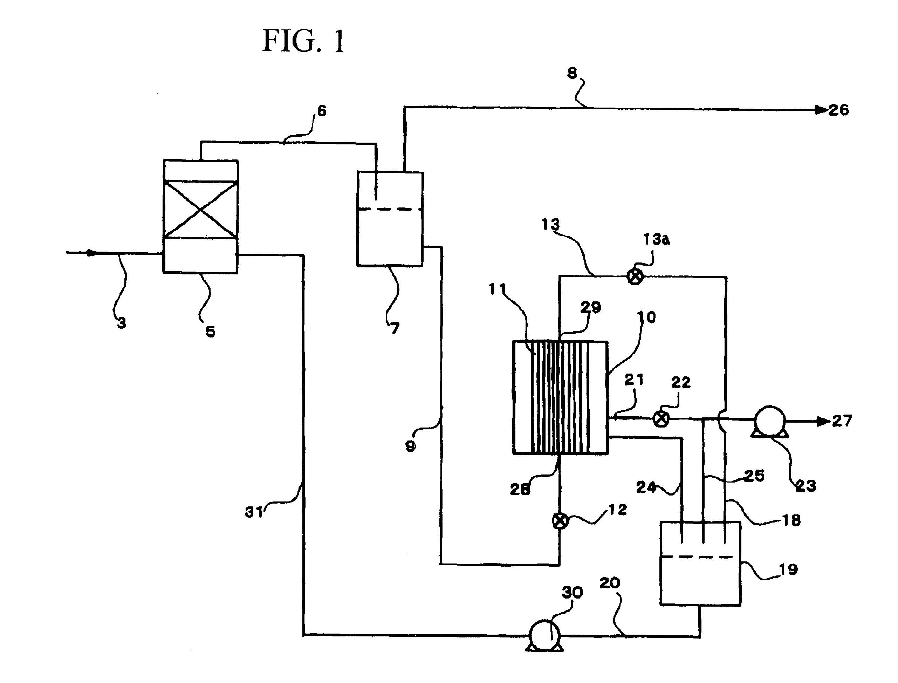

FIG. 1 shows a schematic configuration of a methane separation apparatus, which is an embodiment of a one stage gas/liquid separation system employing the membrane/absorption hybrid method. This methane separation apparatus includes a mixer 5, a first gas/liquid separator 7, and a membrane module 10. The mixer 5 mixes a biogas containing methane and carbon dioxide as its components with an absorbing liquid that absorbs carbon dioxide to form a mixed fluid, which is in a gas-liquid mixed phase. In the first gas/liquid separator 7, the mixed fluid is introduced so as to separate it through gas/liquid separation into methane and a CO₂-absorbed liquid formed of the absorbing liquid and carbon dioxide absorbed therein. The membrane module 10 is comprised of a plurality of hollow fiber permeable membranes 11 built in a container and separates carbon dioxide by supplying the CO₂-absorbed liquid to the inside of the permeable membranes via a supply port 28 so as to permeate the permeable membranes 11 while releasing the carbon dioxide in the CO₂-absorbed liquid to the outside of the permeable membranes 11 due to the lowering of the pressure outside the permeable membranes 11 down to a level lower than that inside the permeable membranes.

In this methane separation apparatus, carbon dioxide is recovered to a carbon dioxide recovery unit 27 by an exhaust passage 21, an on/off valve 22, and an exhaust pump 23. The CO₂-absorbed liquid will become the absorbing liquid after releasing carbon dioxide to be discharged from the membrane module 10 and then recovered and stored in an absorbing liquid storage tank 19 via a recovery passage 24. In addition, the excessive CO₂-absorbed liquid discharged from a discharge port 29 of the membrane module is recovered and stored in the absorbing liquid storage tank 19 via a discharge passage 13, an on/off valve 13a, and a recovery passage 18. The first separation unit that recovers the absorbing liquid after separating carbon dioxide therefrom in the present invention is configured from the membrane module 10 and the absorbing liquid storage tank 19.

Biogas is supplied to the mixer 5 via a supply passage 3. In addition, the absorbing liquid recovered by the absorbing liquid storage tank 19 is supplied cyclically to the mixer 5 by an introduction pump 30 via supply passages 20 and 31 to configure an absorbing-liquid circulation system as a whole.

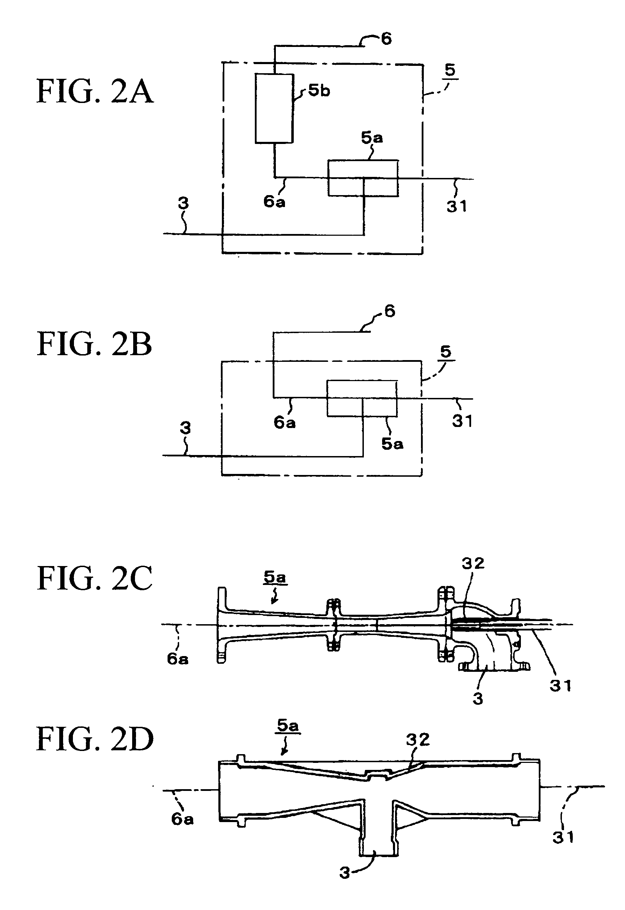

FIG. 2 shows a schematic configuration of the mixer 5. In the drawing of FIG. 2A, the mixer 5 is configured by arranging an ejector 5a and a packed bubble column 5b in series. The biogas supplied from a biogas supply passage 3 and the absorbing liquid supplied from a supply passage 31 are mixed in the ejector 5a and a mixed fluid in a gas-liquid mixed phase where the biogas is mixed in the absorbing liquid in the form of numerous minute air bubbles is delivered to the packed bubble column 5b from a delivery passage 6a. Further stirring of the gas-liquid mixed phase is performed in the packed bubble column 5b and the carbon dioxide in the biogas dissolves in the absorbing liquid due to this two stage operation. The mixed fluid is delivered from a flow path 6 after the carbon dioxide is separated from methane, which is in a gaseous state. Particularly in an apparatus configuration where the aforementioned packed bubble column is provided in the wake side of a gas mixing/merging section of the ejector 5a, the carbon dioxide in the biogas can be absorbed by the absorbing liquid at an even higher percentage and methane can be separated at a higher efficiency. In the drawing of FIG. 2B, the mixer 5 is configured merely from the ejector 5a. It has become apparent from the present invention that satisfactory dissolution of carbon dioxide in the absorbing liquid takes place and the streamlining of methane separation can be achieved by the gas-liquid mixing operation by the ejector 5a alone. In this case, the mixed fluid is delivered from the delivery passage 6a to the flow path 6.

Drawings of FIG. 2C and FIG. 2D in FIG. 2 are cross sectional diagrams of 2 kinds of ejectors 5a. Needless to say, an ejector 5a having other configurations may be used in the present invention. The gas-liquid mixing operation by the ejector 5a will be described in detail below. A nozzle 32 that is sharply narrowed down is formed inside the ejector 5a. The absorbing liquid is supplied from the supply passage 31 to the ejector 5a by the introduction pump 30. The absorbing liquid is jetted from the nozzle 32 at high speed and this formation of high speed flow generates a negative pressure inside the biogas supply passage 3. Effects of the negative pressure increases as the velocity of the aforementioned high speed flow increases and the biogas supplied at a supply pressure that is slightly higher than the atmospheric pressure is sucked by the absorbing liquid and instantly turns into a form of minute gas bubbles, and thus formation of the mixed fluid can simply be carried out. Due to the formation of numerous minute gas bubbles, the gas/liquid contacting surface area with the absorbing liquid increases dramatically and the carbon dioxide in the minute gas bubbles rapidly dissolves in the absorbing liquid. The carbon dioxide is separated from methane, which is in a gaseous state, through gas separation and the aforementioned minute gas bubbles will become minute gas bubbles of methane. A gas-liquid two-phase flow composed of the minute gas bubbles of methane and the absorbing liquid in which carbon dioxide is dissolved is delivered from the delivery passage 6a. Note that a plurality of absorbing-liquid supply passages 31 may be provided concomitantly although only one absorbing-liquid supply passage 31 is shown in FIG. 2.
In addition, it is not limited to the gas mixing/merging section of an ejector system shown in FIG.2 and other fluid merging mechanisms having equivalent performance may be used in the mixer 5. When the ejector system shown in FIG. 2 is adopted as the mixer 5, negative pressure is generated automatically inside the biogas supply passage 3 due to hydrodynamics. Since biogas is sucked by the absorbing liquid due to the effects of this negative pressure, a biogas blower (not illustrated) that is usually installed in the biogas supply passage is no longer required, and thus further reduction in the power of an apparatus can be achieved.

The mixed fluid formed in the mixer 5 is introduced to the first gas/liquid separator 7 via the flow path 6. The first gas/liquid separator 7 separates the mixed fluid through gas/liquid separation into methane and a CO₂-absorbed liquid formed due to an absorption of CO₂ by the absorbing liquid and stores them. The methane separated in this process is emitted from an exhaust pump (not illustrated) via a recovery passage 8 and is recovered by a methane recovery unit 26. The CO₂-absorbed liquid stored in the first gas/liquid separator 7 is transferred to the membrane module 10 via a supply passage 9 due to the gravitational effect caused by the height difference between the first gas/liquid separator 7 and the membrane module 10 or due to the drive of supply pump (not illustrated). It is configured so that the amount of CO₂-absorbed liquid transferred is adjustable by an on/off valve 12 provided in the supply passage 9.

The CO₂-absorbed liquid from the first gas/liquid separator 7 is introduced inside a permeable membrane and permeates a permeable membrane 11. By making the pressure outside the permeable membrane 11 lower than that inside the permeable membrane 11 due to the evacuation inside the membrane module 10 using an exhaust pump 23 for lowering pressure via an exhaust passage 21 and an on/off valve 22, the carbon dioxide in the CO₂-absorbed liquid is released to the outside of the permeable membrane 11 to achieve the separation of carbon dioxide. The separated carbon dioxide is recovered by a carbon dioxide recovery unit 27 via an exhaust passage 21. On the other hand, the CO₂-absorbed liquid turns, after separating carbon dioxide therefrom, into the absorbing liquid. The liquid is then recovered by the absorbing liquid storage tank 19 via a recovery passage 24, supplied again from the absorbing liquid storage tank 19 to the mixer 5, and recycled.
The excessive CO₂-absorbed liquid discharged from the discharge port 29 of the membrane module 10 is recovered by the absorbing liquid storage tank 19 via the discharge passage 13, the on/off valve 13a, and the recovery passage 18. The carbon dioxide released in the absorbing liquid storage tank 19 is recovered by the carbon dioxide recovery unit 27 via a recovery passage 25. Note that the on/off valve 13a is provided so as to retain the permeable membrane 11 in a liquid-sealed state and a system where pipe diameter is narrowed down, for example, a restricted orifice may also be used.

As described above, in the present embodiment, carbon dioxide is separated by supplying the CO₂-absorbed liquid to the membrane module 10 after recovering the methane separated in the first gas/liquid separator 7. Moreover, the excessive CO₂-absorbed liquid discharged from the membrane module 10 is recovered by the absorbing liquid storage tank 19 and is separated into carbon dioxide and the absorbing liquid by releasing carbon dioxide. Accordingly, the separation of methane and carbon dioxide can be carried out efficiently in the circulation process of the absorbing liquid and methane can be separated/purified at high efficiency from the biogas containing high concentrations of carbon dioxide. Therefore, it is possible to achieve a methane separation system that is capable of reducing power load or membrane module cost and can carry out the separation/concentration of biogases at a low separation cost.

In the present embodiment, it is preferable to use an aqueous solution of diethanolamine (DEA) having excellent properties in absorbing carbon dioxide as the absorbing liquid. DEA can be used at a concentration of 0.1 to 6 mol/L (preferably 2 to 4 mol/L).Absorption properties and release properties of carbon dioxide are satisfactory within this DEA concentration range and methane can be separated/purified from biogas at high efficiency.

According to the verification by the present inventors, the release efficiency of carbon dioxide per biogas treatment flow rate does not improve unless the permeation flow rate of the CO₂-absorbed liquid permeating the permeable membrane reaches a predetermined value or more. Therefore, regeneration of the absorbing liquid recovered by the absorbing liquid storage tank 19 via the recovery passage 24 will be unsatisfactory. As a result, when the biogas supplied to the mixer 5 and the absorbing liquid to be recycled are mixed, the absorption capacity of the absorbing liquid towards the carbon dioxide in biogas declines compared to the case where the absorption capacity of the absorbing liquid is restored sufficiently. In other words, methane concentration in the gas recovered in the first gas/liquid separator 7 does not increase. In addition, it has become apparent that when the permeation flow rate of liquid per membrane area is low, regeneration of the absorbing liquid will be unsatisfactory unless an excessive membrane module (in terms of area) is used, and the concentration of methane does not increase. Accordingly, by using the membrane module 10 capable of making the permeation flow rate of the CO₂-absorbed liquid permeating the permeable membrane 11 at 5 to 50 L/m²·min per membrane area (the permeation flow rate of the CO₂-absorbed liquid is preferably 20 to 40 L/ m²·min per membrane area), the release properties of carbon dioxide improve and the enhancement of purified methane concentration is achieved.

Note that when a state of so-called liquid exhaustion where the flow rate of the CO₂-absorbed liquid is reduced and the liquid membrane will become thin in the upper part of the membrane module is brought about, it is necessary to give sufficient consideration since it is possible to result in a decline in the methane recovery rate by the gas permeation (supply of the product gas will be impossible in pronounced cases) in the case of introducing gas/liquid mixture (that is, a simultaneous introduction of methane and the absorbing liquid).In addition, in the case where gas and liquid are separated (introducing only the absorbing liquid into the membrane module), it is necessary to take caution since it is possible that the methane recovery rate declines due to the suction (back flow) of the product gas in, for example, a system where the absorbing liquid side in the membrane is connected to the product gas line, when the membrane is not used effectively.

In addition, it has become apparent due to the verification of the present inventors that at the time of the release of carbon dioxide in the regeneration of the absorbing liquid, congestion of the membrane module prevents the release. In other words, the packing density of permeable membranes affects the performance of carbon dioxide release in the membrane module. The packing density of permeable membranes in the commercially available membrane modules is 30 to 70% and the interval between adjacent permeable membranes is too packed. For this reason, space between membranes will be covered with liquid membranes when the liquid flow rate is large which makes the release efficiency of carbon dioxide more impaired as it approaches the center due to the reduced pressure. As a result, a large membrane area will be required causing a cost increase.
Accordingly, in the present embodiment, the release properties of carbon dioxide are enhanced and the methane separation at high efficiency is achieved by using the membrane module 10 where the packing density of permeable membranes is 30% or less (preferably 20% or less) which is sparse. In addition, it is preferable that the hollow fiber permeable membrane in the membrane module be segmented into small bundles for arrangement and each of the small bundle be arranged so as to retain uncongested space therebetween, and a packing density as a whole be 30% or less. Due to this configuration, it will be possible to further enhance the release properties of carbon dioxide and to separate methane at high efficiency.

Polyethylene is preferable for the material of the permeable membrane 11 and highly efficient processing of the methane separation/purification will be possible especially when the outer surface of the membrane is subjected to a hydrophilic treatment. Concerning the quality of membrane materials, when membrane materials such as polysulfone (PS: often impossible to adjust membrane densities depending on the manufacturer), polyethersulfone (PES), and polyethylene (PE) were tested, satisfactory results are obtained with PES and PS. However, since PES swells with time upon contact with diethanolamine (DEA) as the absorbing liquid and the reductions in the flow rate of permeating liquid and the biogas separation performance have been observed, it is preferable to select polyethylene for practical reasons.
In other words, by using a hydrophobic polyethylene membrane as the permeable membrane, separation selectivity, permeation rate and long term stability improve compared to those of the conventional permeable membranes. Accordingly, the resistance to, for example, diethanolamine as the absorbing liquid, substantially required permeation amount of the absorbing liquid, and economic efficiency can be improved dramatically. However, since the polyethylene membrane is hydrophobic, when the apparatus operation is stopped without filling the membrane module with the absorbing liquid, the wetting of outer surface of the permeable membrane with the absorbing liquid will be unsatisfactory when relaunching the apparatus operation which results in a possible decline of separation efficiency. However, in the present invention, the decline in separation efficiency at the time of launching the apparatus operation can be prevented by subjecting only the outer surface of the permeable membrane to a hydrophilic treatment chemically or by carrying out a physical treatment to form a surface with microscopic asperities for enhancing affinity with the absorbing liquid.

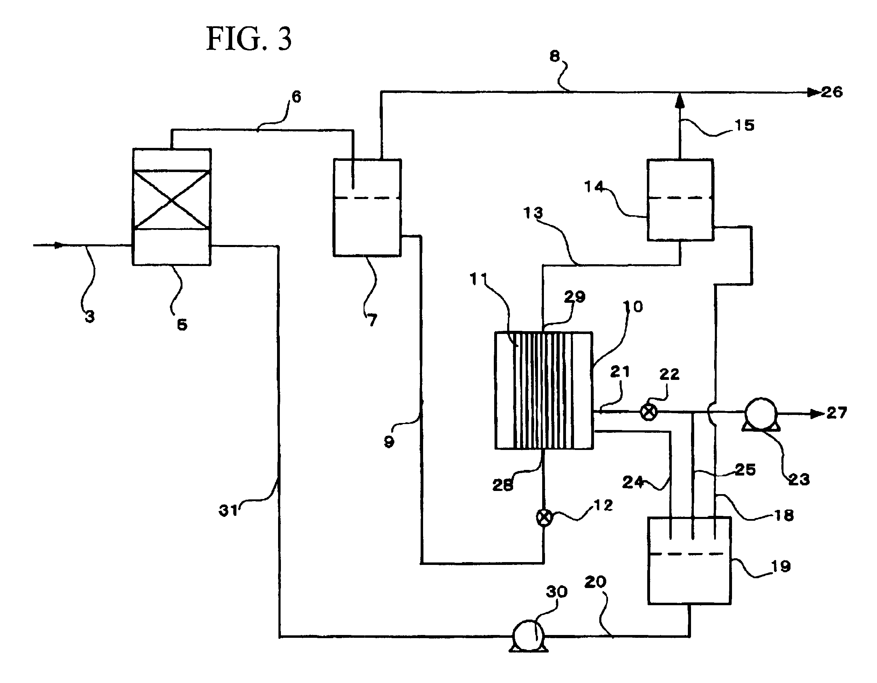

FIG. 3 shows a schematic configuration of a methane separation apparatus, which is an embodiment of a two stage gas/liquid separation system employing the membrane/absorption hybrid method. This methane separation apparatus is different from the methane separation apparatus shown in FIG. 1 by having an additional second gas/liquid separator 14. The discharge passage 13 is connected to the second gas/liquid separator 14 and a trace amount of methane contained in the excessive CO₂-absorbed liquid is separated and recovered by the methane recovery unit 26 via a recovery passage 15. The excessive CO₂-absorbed liquid from which a trace amount of methane is separated is recovered by the absorbing liquid storage tank 19 via the recovery passage 18. The constituting members already shown in FIG. are shown here with the same reference symbols. Since the function/effects of the constituting members shown here with the same reference symbols are exactly the same as those shown in FIG. 1, detailed descriptions thereon will be omitted and only essential points will be described.

In the present embodiment, carbon dioxide is separated by supplying the CO₂-absorbed liquid to the membrane module 10 after recovering the methane separated in the first gas/liquid separator 7. Moreover, the excessive CO₂-absorbed liquid discharged from the membrane module 10 is introduced to the second gas/liquid separator 14 to recover the separated methane. Then the excessive CO₂-absorbed liquid is recovered by the absorbing liquid storage tank 19 and is separated into carbon dioxide and the absorbing liquid by releasing carbon dioxide. Accordingly, the separation of methane and carbon dioxide can be carried out efficiently in the circulation process of the absorbing liquid and methane can be separated/purified at high efficiency from the biogas containing high concentrations of carbon dioxide. As a result, it is possible to achieve a methane separation system that is capable of reducing power load or membrane module cost and can carry out the separation/concentration of biogases at a low separation cost.

Next, an embodiment of a methane utilization system according to the present invention will be described.

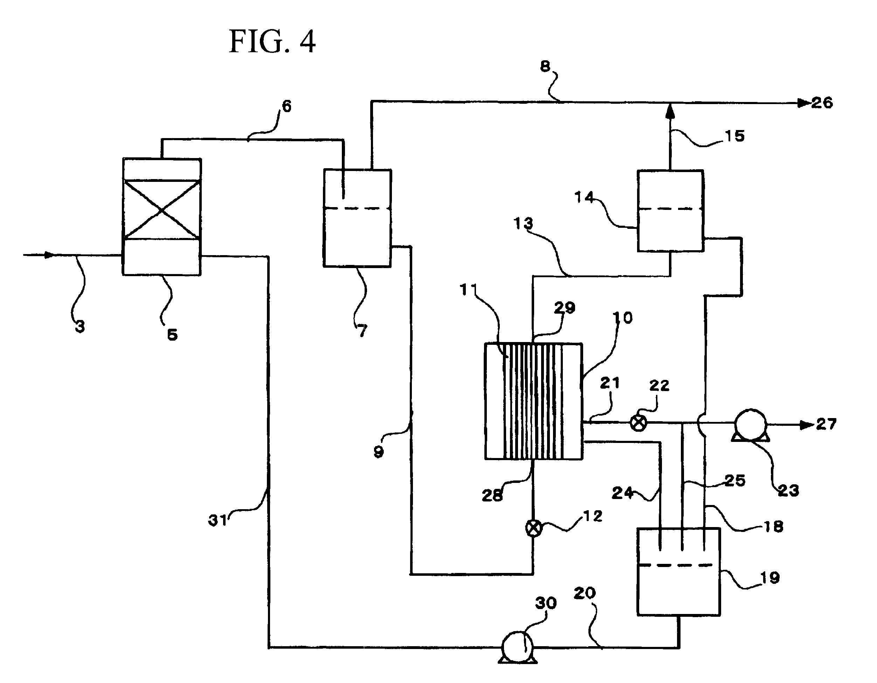

FIG. 4 shows a schematic configuration of a methane utilization system 100 in which the methane separation apparatus according to the present invention is incorporated. This methane utilization system 100 includes power generators 52, 61, and 63 that generate power by using methane as a fuel and it is configured so that the power generated by the power generators can be supplied and sold for users. Methane is separated/purified from the biogas supplied from a biogas fermentation tank 51 using a membrane/absorption hybrid apparatus 50, which is the same methane separation apparatus as that in the abovementioned embodiment. The power generated by the power generator 52 is also used for driving the respective constituting elements of the system. The purified methane obtained from the membrane/absorption hybrid apparatus 50 is supplied to the power generators 61 and 63 by a supply pump 66 via a calorie controller 67 and a supply passage 64. In addition, the purified methane is supplied to liquefaction equipment 56 via a supply passage 65 and the liquefied methane is stored in a liquefied methane storage tank 57. It is configured so that the liquefied methane can be supplied to the outside of the system via an external supply passage 59. In addition, it is configured so that the liquefied methane can also be supplied to the power generators 61 and 63. Flow rate controllers 54, 55, 58, 60, and 62 are provided to each supply passage. A hot water supply mechanism (not illustrated) is provided in the membrane/absorption hybrid apparatus 50 and the supply of hot water by the hot water supply mechanism is carried out in a hot water storage tank 53, in which the heating by a heater is controlled by the power generator 52.
In the methane utilization system having the abovementioned configuration, electric power is generated by the power generators 52, 61, and 63 using the methane separated/purified at high efficiency by the methane separation apparatus according to the present invention as a fuel, and the generated power is supplied to users via repeaters 81 and 82 and a power line 80.

As an operation example of the abovementioned methane utilization system 100, it is possible to optimize the operating rate of the power generators 52, 61, and 63 by adjusting the storage level of the liquefied methane storage tank 57 due to the flow rate control by the flow rate controllers 54, 55, 58, 60, and 62 while taking electric power selling price (for example, 9 Japanese Yen / kWh during the day time from 8:00 to 20:00 and 4 Japanese Yen / kWh during the night time from 20:00 to 8:00 next morning) into consideration. Moreover, specific operation examples will be shown below.

(1) Example of operating rate control of power generator depending on fluctuations in electric power selling price
   The power generator 52 is operated constantly while the power generators 60 and 62 are operated during the day time from 8:00 to 20:00 in order to lower the storage rate as much as possible. During the night time from 20:00 to 8:00 next morning, the power generators 60 and 62 stop operation and the stored fraction is used for running the maximum operation.
(2) Example of absorption control of seasonal fluctuations in amount of biogas generation
   The amount of generated biogas fluctuates greatly due to the fluctuations in the average temperature. For example, since the amount of biogas generation is large in summer and small in winter, it may be controlled so as to store a large amount during summer and the fraction stored during summer is used in winter.
(3) Example of absorption control of fluctuations in biogas calorie
   Gas calorie is calculated by measuring methane concentration using a gas concentration meter 68 provided in the side where methane is discharged. It is possible to stabilize fuel quality by supplying liquefied petroleum gas (LPG) from an LPG tank 69 to the calorie controller 67 depending on the calculation results to add to the purified methane, and controlling the amount to be added.

As described so far, a methane utilization system capable of purifying and storing methane based on the highly efficient methane separation method of the present invention and supplying the stored methane as a fuel can be constructed. In addition, a power supply system capable of stably supplying the electric power generated by the power generation facility to the outside of the system by efficiently adjusting the stored amount of purified methane depending on the season and the time of day and using methane as a fuel can be achieved.

In the abovementioned methane utilization system 100, a carbon dioxide utilization system 101 that uses the carbon dioxide produced as a byproduct during the methane separation/purification is provided concomitantly. In FIG. 4, the recovered carbon dioxide obtained from the membrane/absorption hybrid apparatus 50 is supplied to liquefaction equipment 72 by a supply pump 70 via a recovery passage 83 and the carbon dioxide is liquefied. The liquefied carbon dioxide is stored in a liquefied carbon dioxide storage tank 73. In addition, it is configured so that the liquefied carbon dioxide can be supplied to user's greenhouse facilities 77 and 79 from the liquefied carbon dioxide storage tank 73. Moreover, it is configured so that the liquefied carbon dioxide can be supplied to the outside of the system via an external supply passage 75. Flow rate controllers 71, 74, 76, and 78 are provided to each supply passage. Stable supply of carbon dioxide will be possible by the control due to the adjustments of the flow rate controllers 71, 74, 76, and 78 so as to supply carbon dioxide to the plants in the greenhouse during the daytime when photosynthetic activities are high directly from the liquefied carbon dioxide storage tank 73 and the membrane/absorption hybrid apparatus 50 and to stop the supply to the greenhouse and stockpiles carbon dioxide in the liquefied carbon dioxide storage tank 73 during the nighttime while monitoring the remaining amount of carbon dioxide in the liquefied carbon dioxide storage tank 73.

Due to the carbon dioxide supply system described so far, efficient use of the carbon dioxide produced as a byproduct can be achieved. Note that it will also be possible to construct a combined gas supply system capable of supplying carbon dioxide as an industrial gas via a carbon dioxide supply facility to different supply destinations.

### EXAMPLES

The present invention will be described below in further detail using Examples.
However, the present invention is not limited to these Examples.

### (Example 1)

In Example 1, comparison test of the concentration of methane concentrated by mixers of 3 different kinds of absorption systems was carried out by using the methane separation apparatus of a one stage separation system shown in FIG. 1.

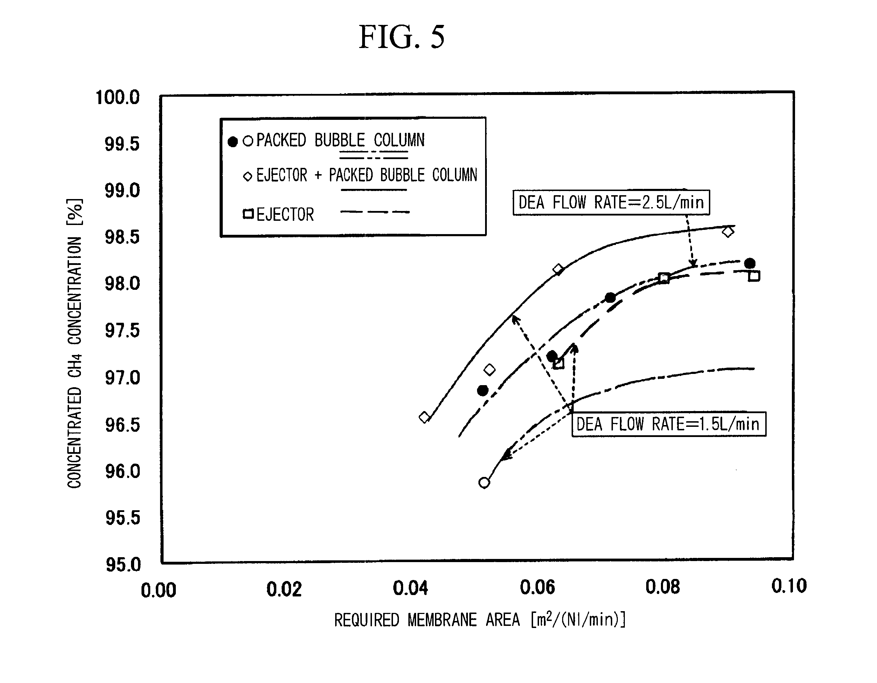

FIG. 5 is a comparison chart of concentrations of the concentrated methane obtained by conducting methane separation from the absorbing liquid that absorbed carbon dioxide using mixers of 3 different absorption systems. The longitudinal axis indicates the concentration of concentrated CH₄ (%) that is separated. The transverse axis indicates the required membrane area (m²/(N1/min)), which is the surface area of a permeable membrane per unit flow rate of a biogas to be treated. More specifically, the required membrane area is defined by the following equation: Required membrane area = (surface area of permeable membrane installed in membrane module) [m²] / (biogas treatment flow rate) [N1/min], and the lower value of required membrane area means higher performance of the absorbing liquid absorbing carbon dioxide, in other words, higher methane separation performance.
The 3 absorption systems refer to the absorption systems of biogas into the absorbing liquid by the mixers and they are a system formed only of a packed bubble column (shown with a symbol • and an alternate long and two short dashes line and a symbol ○ and an alternate long and short dash line), a system where an ejector and a packed bubble column are arranged in series (shown with a symbol 0 and a solid line), and a system formed only of an ejector (shown with a symbol □ and a dashed line). The solid line, the dashed line, and the alternate long and short dash line show the results obtained at a DEA flow rate of 1.5 (L/min) and the alternate long and two short dashed line shows the results obtained at a DEA flow rate of 2.5 (L/min). The results indicate that the mixers with different carbon dioxide absorption methods will require different membrane module areas for obtaining a product with the same methane concentration.

As is apparent from FIG. 5, the system where the ejector and the packed bubble column are arranged in series (shown with the symbol 0 and the solid line) will result in the absorbing liquid with the highest carbon dioxide absorption performance, and thus methane can be recovered at a high purity and a high recovery rate by the gas/liquid separator situated in the wake side. However, the apparatus will be large in size and the apparatus price will be high when this system is employed, and thus the advantage of employing the system will be reduced. In addition, although it appears the stand alone packed bubble column (shown with the symbol • and the alternate long and two short dashes line) and the stand alone ejector (shown with the symbol □ and the dashed line) will result in almost the same extent of carbon dioxide absorption performance, it could be judged that the stand alone ejector is superior for the following reasons.
It is apparent that the system employing the stand alone packed bubble column cannot achieve the required methane concentration unless the amount of absorbing liquid supplied is raised up to 1.5 fold or more than that of the system employing the stand alone ejector. That is, the results of the stand alone packed bubble column shown with the alternate long and two short dashes line (•) are obtained at a DEA flow rate of 2.5 (L/min) whereas the results of the stand alone ejector shown with the dashed line (□) are obtained at a DEA flow rate of 1.5 (L/min). Therefore, carbon dioxide absorption performance is higher and the methane separation can be carried out at a higher efficiency with the system employing the stand alone ejector than the system employing the stand alone packed bubble column. Since the production of gas-liquid mixed phase can be achieved naturally due to the hydrodynamic effects with the stand alone ejector, it could be judged that it is even more effective also for the reason of power cost reduction. Therefore, the methane separation apparatus can be provided that is also excellent in terms of cost reduction since the system with the stand alone ejector does not require a large scale packed bubble column.
Note that highly pure methane was not obtained with the stand alone packed bubble column (shown with the symbol ○ and the alternate long and short dash line) where the DEA flow rate was 1.5 (L/min). In addition, this graph can be used for calculating the apparatus outlines (that is, a permeable membrane area required for the membrane module) as long as the specifications of methane separation apparatus (for example, flow rate of the biogas to be treated, concentrations of the contained impurities, flow rate and concentration of the recovered methane, and the like) are determined.

### (Examples 2 to 5)

In Examples 2 to 5, separation and purification of methane were carried out using the methane separation apparatus of a one stage separation system shown in FIG. 1.
Tables 1 to 4 show details of the respective conditions, in which Examples 2 to 5 were conducted.

**[Table 1]**

| | | | | | | |
|---|---|---|---|---|---|---|
| Biogas treatment flow rate | Nl/min | 0.67 | Degree of vacuum | kPaG | | -90.2 |
| CH₄ concentration | % | 98.4 | DEA temperature | °C | | 29 |
| CH₄ recovery rate | % | 99.5 | Membrane area | m² | | 0.062 |
| Required membrane area | m²/(Nl/min) | 0.09 | Flow rate of permeating liquid per membrane area | L/m²·min | | 40.6 |
| Gas/liquid ratio (Nl/L) | 0.27 | | Absorbing liquid | | 3 mol/l-DEA | |
| Hollow fiber material | Polyethylene φ0.7 mm | | Membrane effective length [cm] | | 47 | |
| Pore size [nm] | 250 | | Membrane filling rate [m²/ m²] | | 0.096 | |

**[Table 2]**

| | | | | | | |
|---|---|---|---|---|---|---|
| Biogas treatment flow rate | Nl/min | 0.67 | Degree of vacuum | kPaG | | -91.3 |
| CH₄ concentration | % | 98.2 | DEA temperature | °C | | 29 |
| CH₄ recovery rate | % | 99.6 | Membrane area | m² | | 0.062 |
| Required membrane area | m²/(Nl/min) | 0.09 | Flow rate of permeating liquid per membrane area | L/m²·min | | 28.4 |
| Gas/liquid ratio (Nl/L) | 0.27 | | Absorbing liquid | | 3 mol/l-DEA | |
| Hollow fiber material | Polyethylene φ0.7 nm | | Membrane effective length [cm] | | 47 | |
| Pore size [nm] | 250 | | Membrane filling rate [m²/ m²]) | | 0.096 | |

**[Table 3]**

| | | | | | | |
|---|---|---|---|---|---|---|
| Biogas treatment flow rate | Nl/min | 0.99 | Degree of vacuum | kPaG | | -89.7 |
| CH₄ concentration | % | 98.2 | DEA temperature | °C | | 36 |
| CH₄ recovery rate | % | 99.7 | Membrane area | m² | | 0.10 |
| Required membrane area | m²/(Nl/min) | 0.10 | Flow rate of permeating liquid per membrane area | L/m²·min | | 7.4 |
| Gas/liquid ratio (Nl/L) | 0.65 | | Absorbing liquid | | 3 mol/1-DEA | |
| Hollow fiber material | Polyethersulfone φ0.8 mm | | Membrane effective length [cm] | | 100 | |
| Pore size [nm] | 10 (molecular weight cutoff 150,000) | | Membrane filling rate [m²/ m²] | | 0.13 | |

**Table 4]**

| | | | | | | |
|---|---|---|---|---|---|---|
| Biogas treatment flow rate | Nl/min | 1.81 | Degree of vacuum | kPaG | | -89.0 |
| CH₄ concentration | % | 93.0 | DEA temperature | °C | | 36 |
| CH₄ recovery rate | % | 99.9 | Membrane area | m² | | 0.175 |
| Required membrane area | m²/(Nl/min) | 0.10 | Flow rate of permeating liquid per membrane area | L/m²·min | | 2.3 |
| Gas/liquid ratio (Nl/L) | 1.20 | | Absorbing liquid | | 3 mol/l-DEA | |
| Hollow fiber material | Polyethersulfone φ0.8 mm | | Membrane effective length [cm] | | 27 | |
| Pore size [nm] | 5 (molecular weight cutoff 30,000) | | Membrane filling rate [m²/ m²]) | | 0.063 | |

First, Tables 1 and 2 show variations in the concentration of separated methane and the methane recovery rate when the flow rate of liquid permeating the hollow fiber permeabel membrane, which had a required membrane area of 0.09m²/(NL/min-biogas) and whose membrane material was polyethylene (PE), was changed. Results are those of Examples conducted when the flow rates of liquid permeating the permeable membrane were 40.6 [L/m²/min], respectively and the methane recovery rate were almost 100% and the concentrations of methane were also 98.4% and 98.2%. Tables 3 and 4 show variations in the concentration of separated methane and the methane recovery rate when the flow rate of liquid permeating the permeable membrane, which had a required membrane area of 0.1 m²/(NL/min-biogas) and whose membrane material was polyethersulfone (PES), was changed. The obtained results show that the concentration of methane increased as the flow rate of liquid permeating the membrane increased. This implies that it is efficient to increase the flow rate of permeation liquid per membrane area and to raise the regeneration efficiency of absorbing liquid due to a pressure reducing operation in order to release the carbon dioxide that is absorbed by the absorbing liquid at high efficiency.
In the Examples shown in Tables 3 and 4, although the methane recovery rate was 99.7 to 99.9% in all of the conditions examined, the methane concentration was within a range of 93.0% to 98.2%, and thus there were cases where the methane concentration was not necessarily high depending on the conditions. On the other hand, in the Examples shown in Tables 1 and 2, the methane recovery rate was 99.5 to 99.8% in all of the conditions examined and the methane concentration remains within a range of 98.2% to 98.4% which suggested that the methane separation apparatus according to the present embodiment is capable of performing highly efficient recovery of highly concentrated methane.

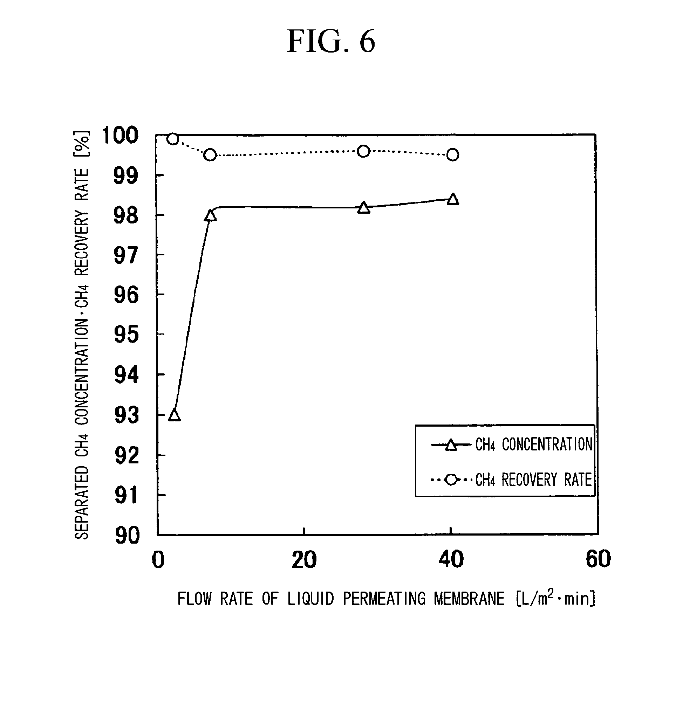

FIG. 6 shows the result of studies conducted in the respective conditions described in Tables 1 to 4 as the relationship among the flow rate of liquid permeating a membrane, the methane recovery rate, and the methane concentration. From FIG. 6, it is apparent that satisfactory performance is achieved in terms of both the methane recovery rate and the methane concentration when the flow rate of liquid permeating a membrane is 5 [L/ m²·min] or more.

### (Example 6)

In Example 6, the methane separation apparatus according to the present invention was compared with the apparatus of a conventional methane purification system in terms of gas separation performance, purification cost, and the like.
Table 5 compares the methane separation apparatus according to the present invention with the apparatus of a conventional methane purification system in terms of gas separation performance, purification cost, and the like.

**[Table 5]**

| Comparison of each system | | | | |
|---|---|---|---|---|
| Item | PSA process | Dry membrane separation process | Chemical absorption method (diethanolamine) | Membrane/absorption hybrid method |
| Operating pressure | Normal pressure (desorption -90 kPaG) | 0.6 to 0.7 MPaG | Normal pressure | Normal pressure (desorption -90 kPaG) |
| CH₄ concentration | 90% | 90% | 90% | 90% |
| CH₄ recovery rate | 90% (CH₄ concentration 90%) | 70% (CH₄ concentration 90%) | ≈100% (no dependence on CH₄ concentration) | ≈100% (no dependence on CH₄ concentration) |
| Required power [kW] | 21 | 20 | 20 | 17 |
| Power unit consumption [kWh/m³] | 0.39 | 0.48 | 0.33 | 0.28 |

| | | | | |
|---|---|---|---|---|
| N.B.) Calculated by assuming the unit cost of electric power of 12 Japanese Yen / kWh, unit cost of industrial water of 35 Japanese Yen / ton, and annual operating time of 8,600 hr. | | | | |

In this table, each methane purification system was compared under the conditions where the composition of the source gas consisted of methane (60 vol.%) and carbon dioxide (40 vol.%) and the flow rate of the source gas was 100 m³/hr.

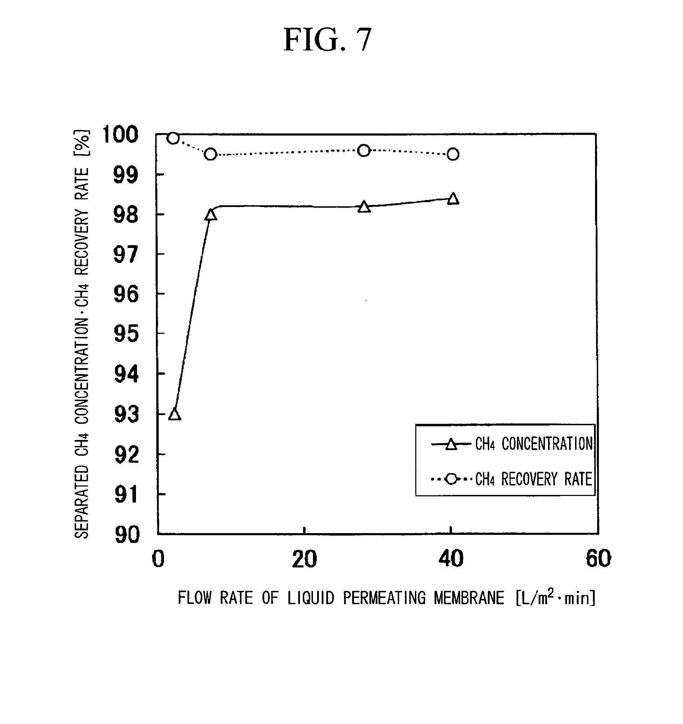

FIG. 7 is a graph showing the relationship among the flow rate of liquid permeating a membrane, the cost for methane separation (in comparison with a conventional case), and the pumping power for the absorbing liquid (kW). The pumping power for the absorbing liquid (kW) increased due to the increase in the flow rate of liquid permeating the membrane. According to the membrane/absorption hybrid method of the present invention for methane separation, the cost for methane separation reduced compared to the conventional separation method when the flow rate of liquid permeating the membrane was 15 to 60 [L/ m²·min]. Note that the practicable and effective flow rate of liquid permeating the membrane was 5 to 60 [L/ m²·min] since, in terms of performance, methane can be separated at high efficiency when the flow rate of liquid permeating the membrane was 5 [L/ m²·min] or more from the results shown in FIG. 6. In addition, FIG. 7 shows even more marked reduction in the separation cost when the flow rate of liquid permeating the membrane was 20 to 40 [L/ m²·min].

It can be said from Table 5 and FIG. 7 that in the separation of a biogas containing high concentration of carbon dioxide, the membrane/absorption hybrid method of the present invention for methane separation and the methane separation apparatus employing the method can separate/purify methane from the biogas at low cost and high efficiency.

Needless to say, the present invention is not limited to the abovementioned embodiments and Examples and includes various modifications, design changes, and the like within the scope, which does not depart from the technical spirit of the present invention, in its technical scope.

### INDUSTRIAL APPLICABILITY

According to the present invention, the methane purification treatment from the biogas having methane as its major component and contains high concentration of carbon dioxide can be conducted at high efficiency as well as at a low separation cost and it is possible to achieve a methane utilization facility and a methane utilization system which can supply purified methane of high purity as an energy source.

## Claims

1. A methane separation method where methane is separated from a biogas containing methane and carbon dioxide as components, the method at least comprising:
mixing the biogas and an absorbing liquid that absorbs carbon dioxide in a mixer so as to form a mixed fluid of a gas-liquid mixed phase;
introducing the mixed fluid into a first gas/liquid separator so as to separate the mixed fluid through gas/liquid separation into methane and a CO₂-absorbed liquid formed due to an absorption of the carbon dioxide by the absorbing liquid;
recovering methane separated in the first gas/liquid separator; and
supplying the CO₂-absorbed liquid through a supply port of a membrane module comprised of a container and a plurality of hollow fiber permeable membranes built therein to inside of the membranes so as to make the CO₂-absorbed liquid permeate the permeable membranes, and lowering a pressure outside the permeable membranes to a level lower than that inside the permeable membranes, thereby releasing the carbon dioxide contained in the CO₂-absorbed liquid to outside of the permeable membranes so as to separate the carbon dioxide, and recovering the absorbing liquid obtained by separating CO₂ from the CO₂-absorbed liquid.

2. The methane separation method according to Claim 1 further comprising:
introducing an excessive CO₂-absorbed liquid discharged from a discharge port of the membrane module to a second gas/liquid separator so as to conduct gas/liquid separation of a trace amount of methane from the excessive CO₂-absorbed liquid;
recovering methane separated in the second gas/liquid separator; and
recovering the excessive CO₂-absorbed liquid.

3. The methane separation method according to Claim 1, wherein a packing density of the permeable membrane in the membrane module is 30% or less.

4. The methane separation method according to Claim 3, wherein
the permeable membrane in the membrane module is segmented into small bundles for arrangement, each of the small bundles is arranged so as to retain uncongested space therebetween, and a packing density as a whole is 30% or less.

5. The methane separation method according to Claim 1, wherein
the mixer comprises an ejector provided in a flow path that communicates to an inlet of the first gas/liquid separator, generates negative pressure by forming a flow of the absorbing liquid inside the flow path, forms the mixed fluid by making the absorbing liquid to suck the biogas, and makes the absorbing liquid to efficiently absorb carbon dioxide.

6. The methane separation method according to Claim 5, wherein
the mixer further comprises a packed bubble column, supplies the mixed fluid formed in the ejector to the packed bubble column, and further accelerates an absorption of carbon dioxide in the mixed fluid.

7. The methane separation method according to Claim 1, wherein the absorbing liquid is an aqueous solution of diethanolamine having a concentration of 0.1 to 6 mol/L.

8. The methane separation method according to Claim 1, wherein a permeation flow rate of the CO₂-absorbed liquid permeating the permeable membrane in the membrane module is 5 to 50 L/ m²·min per membrane area.

9. The methane separation method according to Claim 1, wherein the permeable membrane is formed of polyethylene.

10. The methane separation method according to Claim 1, wherein a membrane outer surface of the permeable membrane is subjected to a hydrophilic treatment.

11. A methane separation apparatus where methane is separated from a biogas containing methane and carbon dioxide as components, the apparatus at least comprising:
a mixer mixing the biogas and an absorbing liquid that absorbs carbon dioxide so as to form a mixed fluid of a gas-liquid mixed phase;
a first gas/liquid separator in which the mixed fluid is introduced so as to separate the mixed fluid through gas/liquid separation into methane and a CO₂-absorbed liquid formed due to an absorption of the carbon dioxide by the absorbing liquid; and
a first separation unit having a membrane module comprised of a container and a plurality of hollow fiber permeable membranes built therein, in which the CO₂-absorbed liquid is supplied through a supply port to inside of the permeable membranes so as to make the CO₂-containing liquid permeate the permeable membranes, and a pressure outside the permeable membranes is lowered to a level lower than that inside the permeable membranes, thereby releasing the carbon dioxide contained in the CO₂-absorbed liquid to outside of the permeable membranes so as to separate carbon dioxide, and recovering the absorbing liquid obtained by separating carbon dioxide from the CO₂-absorbed liquid due to the membrane module.

12. The methane separation apparatus according to Claim 11 further comprising:
a second gas/liquid separator in which an excessive CO₂-absorbed liquid discharged from a discharge port of the membrane module is introduced so as to conduct gas/liquid separation of a trace amount of methane from the excessive CO₂-absorbed liquid; and
a second separation unit recovering the excessive CO₂-absorbed liquid, wherein
separated methane is recovered by the first gas/liquid separator and the second gas/liquid separator.

13. The methane separation apparatus according to Claim 11, wherein a packing density of the permeable membrane in the membrane module is 30% or less.

14. The methane separation apparatus according to Claim 11, wherein
the mixer at least comprises an ejector provided in a flow path that communicates to an inlet of the first gas/liquid separator, a unit that introduces the absorbing liquid, and a unit that introduces the biogas, and generates negative pressure by forming a flow of the absorbing liquid inside the flow path, forms the mixed fluid by making the absorbing liquid to suck the biogas, and makes the absorbing liquid to efficiently absorb carbon dioxide.

15. The methane separation apparatus according to Claim 11, wherein the absorbing liquid is an aqueous solution of diethanolamine having a concentration of 0.1 to 6 mol/L.

16. The methane separation apparatus according to Claim 11, wherein a permeation flow rate of the CO₂-absorbed liquid permeating the permeable membrane in the membrane module is 5 to 50 L/ m²·min per membrane area.

17. The methane separation apparatus according to Claim 11, wherein the permeable membrane is formed of polyethylene.

18. A methane utilization system comprising:
the methane separation apparatus according to Claim 11;
a methane storage tank; and
a methane supply passage; wherein
methane is purified and stored by removing carbon dioxide from at least one of the biogas selected from the group consisting of a natural gas that is generated from underground due to anaerobic fermentation by organisms and has methane as its major component, an underground fermentation gas produced by a natural anaerobic fermentation due to an underground burial of industrial and domestic wastes, and an artificial fermentation gas discharged from an anaerobic fermentation process that is set up artificially, and
the stored methane can be supplied as a fuel.

19. The methane utilization system according to Claim 18, further comprising:
a power generation facility generating electricity using the stored methane as a fuel; and
a storage control unit that adjusts a stored amount of purified methane depending on seasons, an operation period, or a time period, wherein
electric power generated by the power generation facility can be supplied to outside.

20. The methane utilization system according to Claim 18, further comprising a carbon dioxide supply facility enabling hybrid supply of carbon dioxide that is separated simultaneously when methane is purified.
